# EUROPEAN PATENT APPLICATION

(11) **EP 1 826 297 A2**
(43) Date of publication of application: **29.08.2007**
(21) Application number: 07250762.7
(22) Date of filing: 23.02.2007
(51) Int. Cl.: C25D 11/10, C25D 11/26, A61N 1/39

(54) **Anodizing electrolytes using a dual acid system for high voltage electrolytic capacitor anodes**

(30) Priority: 23.02.2006 US 776168 P
(71) Applicant: Greatbatch Ltd., Clarence, NY 14031 (US)
(72) Inventor: Liu, Yanming, Clarence Center NY 14032 (US); Goad, David, Buffalo NY 14213 (US); Budek, David, Amherst NY 14226 (US); Scheuer, Christina, Amherst NY 14228 (US)
(74) Representative: Duckett, Anthony Joseph

(57) **Abstract**

An improved formation electrolyte and method for anodizing valve metal anodes used in electrolytic capacitors, particularly for high voltage sintered tantalum powder anode, is described. The anodizing electrolyte composition is comprised of 1) a phosphorus oxyacid and/or its salt, such as phosphoric acid and ammonium,phosphate; 2) a weak inorganic acid/salt (such as boric acid, ammonium borate) or a weak carboxylic acid/salt; 3) water; and 4) a protic solvent or a mixture of two or more protic solvents. The weak mono-carboxylic acid/salt has 2 to 7 carbon atoms and the weak di- or poly-carboxylic acid/salt has 3 to 13 carbon atoms. The present electrolytes have high anodizing breakdown voltage capability and the formed dielectric oxides have improved oxide quality including good oxide hydration resistant ability, and result in more stable capacitor performance. These properties are particularly important for critical applications such as implantable cardioverter defibrillators (ICDs). Significantly, this means that fewer capacitors are needed to meet an ICD's operating voltage.

## Description

### CROSS-REFERENCE TO RELATED APPLICATION

This application claims priority from U.S. Application Serial No. 60/776,168, filed February 23, 2006.

### BACKGROUND OF THE INVENTION

### 1. Field of the Invention

This invention relates to anodizing electrolytes and methods for anodizing valve metal anodes, particularly for high voltage sintered tantalum powder anodes used in electrolytic capacitors.

### 2. Prior Art

Electrolytic capacitors are well known for use in a variety of electronic equipment such as consumer audio and video equipment, home appliances, power supplies, industrial electronics, military electronics, computers, telecommunication equipment, entertainment equipment, automotive devices, lighting ballasts, and implantable medical devices. In general, electrolytic capacitors comprise an anode and a cathode segregated from each other by at least one layer of separator material impregnated with a working electrolyte. The anode is a valve metal body coated with a layer of the corresponding metal oxide serving as a dielectric.

An implantable cardioverter defibrillator (ICD) operates at high voltages, typically from about 600 volts to about 800 volts, and requires high voltage capacitors. Typically, two to four valve metal capacitors, preferably tantalum capacitors, are connected in series to achieve the desired voltage. Increasing the capacitor working voltage reduces the number of capacitors needed per device. However, development of higher voltage capacitors is, in part, limited by the ability of the anodizing electrolyte to form valve metal anodes to a higher voltage.

From time-to-time, the ICD is required to deliver an electrical shock therapy to the heart to treat tachyarrhythmias, the irregular, rapid heartbeats that can be fatal if left uncorrected. During charging of a capacitor for that purpose, every second is critical for patient survival. Rapid charging requires that the capacitors have a stable non-operating life. Unfortunately, electrolytic capacitors degrade when not in operation. This so called "shelf" instability is worse in aluminum electrolytic capacitors than in tantalum electrolytic capacitors. Nonetheless, degradation is mainly due to hydration of the dielectric oxide by water present in the capacitor working electrolyte. Oxide degradation increases the time of the first charging cycle after an extended non-operation period and reduces the capacitor charge/discharge energy efficiency. This is undesirable for critical applications such as ICDs, in which dielectric oxide degradation increases capacitor (device) charging time and decreases the useful life of the battery or increases battery and device volume.

Only tantalum and aluminum capacitors are currently used in ICDs because their high voltage and high volumetric energy density capabilities. Because ICDs normally operate at from 600 volts to 800 volts and the working voltage of current tantalum and aluminum capacitors ranges from 200 volts to 400 volts, two to four capacitors are used in series for powering an ICD. However, the development of higher voltage capacitors would allow fewer capacitors to be used per ICD device.

Correspondingly, the anodes for higher voltage capacitors need to be formed to higher voltages. But, the ability of high voltage anode formation depends on a number of factors including tantalum powder micro-morphology, powder chemistry, press/sintering conditions, anodizing electrolyte composition, and anodizing protocols. Electrolyte composition, especially the anions (phosphate in case of phosphoric acid and phosphate salts) in electrolytes is a critical factor for successful high voltage anode formation. The phosphoric/polyglycol-based anodizing electrolytes have been used in forming high voltage tantalum anodes. This type of electrolyte consists of phosphoric/phosphate solutes and glycol or polyglycol solvents.

It is observed that the anodic oxide formed in phosphate-based electrolytes contains significant phosphorus incorporated therein. It has been proved that phosphorous improves dielectric oxide hydration resistance and long-term performance stability of the resulting capacitor. However, phosphoric/phosphate solutes have relatively lower anodizing breakdown voltages than some of the weak inorganic (e.g. boric acid and borate) or carboxylic acids. Although lowering the electrolyte conductivity by reducing the phosphoric/phosphate concentration or increasing the solvent content can enhance the anodizing breakdown voltage, the electrolyte eventually becomes impractical when its IR drop becomes too high due to increased electrolyte resistivity.

Certain inorganic acids/salts and weak organic acids/salts have higher formation voltage capability. But, the anodic oxides formed in these electrolyte compositions do not have the oxide hydration resistance and long-term performance stability characteristic of phosphoric/phosphate-based electrolytes.

Therefore, an improved anodizing electrolyte and method of anodization is needed to not only provide for higher voltage anodic oxide formation, but also to improve DC leakage and hydration resistance properties. The present invention provides a mixed anodizing electrolyte composition as well as an improved anodizing method that provide high voltage oxide formation capability while still maintaining low DC leakage and hydration resistance ability.

### SUMMARY OF THE INVENTION

In that respect, this invention has two objectives. The first is to provide an anodizing electrolyte comprised of: 1) a phosphorus oxyacid and/or its salt, such as phosphoric acid and ammonium phosphate; 2) a weaker inorganic acid/salt (such as boric acid, ammonium borate) or a weak mono-carboxylic acid/salt having 2 to 7 carbons or a weak di- or poly-carboxylic acid having 3 to 13 carbons; 3) water; and 4) a protic solvent or a mixture of two or more protic solvents. The second objective is to provide an improved anodizing method for valve metal structures in an anodizing' electrolyte containing phosphorus oxyacids/salts and a weaker inorganic acid/salt (such as boric acid, ammonium borate) or a weak mono-carboxylic acid/salt having 2 to 7 carbons or a weak di- or poly-carboxylic acid having 3 to 13 carbons.

The electrolytes have relatively high anodizing breakdown voltages, which allows for formation of high voltage dielectric oxides, and results in improved dielectric oxide quality. This means that the resulting capacitor has higher operating voltage, lower DC leakage, good hydration resistance and more stable lifetime performance. These properties are particularly important for critical applications such as required by implantable cardioverter defibrillators (ICDs). When used in ICDs, capacitors made with anodes formed according to the present invention, which can be formed to higher formation voltages, allow for fewer capacitors per device, provide shorter first charging time, higher energy efficiency, and more stable lifetime performance.

The foregoing and additional objects, advantages, and characterizing features of the present invention will become increasingly more apparent upon a reading of the following detailed description.

### DETAILED DESCRIPTION OF THE PREFERRED EMBODIMENTS

Although the present invention is focused on sintered tantalum powder anodes, it applies to all valve metals used in electrolytic capacitors such as, but not limited to, tantalum, vanadium, niobium, aluminum, titanium, zirconium, hafnium, and mixtures thereof, in the form of foil (etched or unetched), sintered powder bodies, and like porous structures.

The dielectric oxide on a valve metal in an electrolytic capacitor is normally formed using a technique known as anodizing. Passing an anodic current through a valve metal immersed in an anodizing (formation) electrolyte does this. The thickness of the resulting anodic oxide is proportional to the anodizing voltage. The desired oxide thickness is determined by the capacitor rated voltage and other required properties. For a given dielectric oxide thickness, the volumetric capacitance and energy density of a capacitor are functions of the specific surface area of the valve metal anode. To increase capacitor volumetric energy density, porous valve metal structures are normally used. Examples include an etched aluminum foil for an aluminum capacitor and a pressed and sintered tantalum powder body for a tantalum capacitor. Furthermore, the dielectric oxide quality depends on a number of factors including the type and purity of the valve metal, anode micromorphology, anode size and geometry, electrolyte composition, temperature, and anodizing protocols.

In that light, this invention is directed to anodizing (formation) electrolytes and methods of anodization. The composition of an anodizing electrolyte affects anodizing breakdown voltage and the quality of the formed anodic oxide. An anodic oxide normally contains species incorporated from the anodizing electrolyte during dielectric oxide formation. For example, the outer layer of an anodic oxide formed in phosphoric acid/phosphate-containing electrolytes contains phosphorus. The amount of the incorporated phosphorous depends on the concentration of the phosphoric acid/phosphate in the anodizing electrolytes, temperature, and anodizing current density. Although the incorporated phosphorus slightly decreases the dielectric constant, it benefits the quality of the anodic oxide by lowering DC leakage and improving oxide hydration resistance. Improved hydration resistance helps stabilize the shelf life and long-term performance of the resulting capacitor. Phosphate-based electrolytes, however, have limited anodizing breakdown voltage and are not capable of formation of relatively high voltage anodic oxides.

Certain weaker inorganic acids (e.g., boric acid) and carboxylic acids (e.g., acetic acid, adipic acid, azelaic acid, dodecanedioic acid) have higher anodizing breakdown voltage capability than phosphoric acid/phosphate electrolytes, but the anodic oxide formed in them has higher DC leakage, poor hydration resistance ability, and less stable long-term performance. Boric acid and large carboxylic acids also have limited solubility, especially at lower temperatures.

However, the present invention describes an anodizing electrolyte composition comprised of: 1) a phosphorus oxyacid and/or its salt, such as phosphoric acid and ammonium phosphate; 2) a weak inorganic acid/salt or a weak carboxylic acid/salt; 3) water; and 4) a protic solvent or a mixture of two or more protic solvents. An improved anodizing method for anodizing a valve metal structure in an electrolyte containing phosphorus oxyacids/salts and a weaker inorganic acid/salt (such as boric acid, ammonium borate) or a weak mono-carboxylic acid/salt having 2 to 7 carbon atoms or a weak di- or poly-carboxylic acid having 3 to 13 carbon atoms is also described.

In particular, the mixed acid electrolytes comprise, by weight, from about 5% to about 80% water, up to about 90% of a protic solvent, from about 0.1% to about 15% phosphorus acid or its salts, and from about 0.5% to about 15% of a weak inorganic or organic carboxylic acid, or their mixture, and their salts. The anodizing electrolyte preferably has a conductivity of about 20 µS to about 10,000 µS, more preferably from 100 µS to about 1,000 µS, at 40°C.

The solvent is a protic solvent including one selected from the group consisting of alkylene glycols (examples include, but are not limited to, ethylene glycol, diethylene glycol, triethylene glycol, tetraethylene glycol, propylene glycol, trimethylene glycol, dipropylene glycol, glycerol, 2-methyl-1,3-propanediol, 1,4-butanediol, 2,3-butanediol, 2,2-dimethyl-1,3-propanediol, 2,4-pentanediol, 2,5-hexanediol, and combinations thereof), polyalkylene glycols (examples include, but are not limited to, polyethylene glycols, polypropylene glycols, polyethylenepropylene glycol copolymers, and mixtures thereof), and alkylene or polyalkylene glycol monoethers (examples include, but are not limited to, ethylene glycol methyl ether, ethylene glycol ethyl ether, ethylene glycol butyl ether, diethylene glycol ethyl ether, diethylene glycol methyl ether, diethylene glycol butyl ether, dipropylene glycol methyl ether, tripropylene glycol methyl ether, and mixtures thereof). The solvent preferably has a molecular weight of less than 1000.

The phosphorus oxyacids/salts include, but are not limited to, phosphoric acid, ammonium dihydrogen phosphate, ammonium hydrogen phosphate, sodium phosphates, potassium phosphates, hypophosphoric acid, phosphorous acid, hypophosphorous acid, metaphosphoric acids, and mixtures thereof.

The weak inorganic acids/salts include, but are not limited to, boric acid, sodium borate, ammonium borate, ammonium tetraborate, ammonium pentaborate, and mixtures thereof.

The carboxylic acids include mono-, di-, and poly-carboxylic acids, and mixtures thereof. The mono-carboxylic acids have from 2 to 7 carbons, and have a straight or branched chain or cyclic structures. Examples of mono-carboxylic acids/salts include, but are not limited to, acetic acid, propanoic acid, butyric acid, iso-butyric acid, trimethyl acetic acid, cyclohexanecarboxylic acid, dicyclohexylacetic acid, and mixtures thereof.

The di-carboxylic acids have from 3 to 13 carbons, and have a straight or branched chain or cyclic structure. Examples of di-carboxylic acids/salts include, but are not limited to, malonic acid, glutaric acid, adipic acid, pimelic acid, azelaic acid, brassylic acid, dodecanedioic acid, 1,2-cyclohexanedicarboxylic acid, cyclohexyldicarboxylic acid, methylmalonic acid, dimethylmalonic acid, 2,2-dimethylsuccinic acid, 2-methylglutaric acid, 2,2-dimethylglutaric acid, 3-(tert-butyl)adipic acid, and mixtures thereof.

Examples of poly-carboxylic acids/salts include, but are not limited to, citric acid, tartaric acid, 1,3,5-cyclohexanetricarboxylic acid, and mixtures thereof.

Ammonium salts of the acids discussed above can be produced in situ by reacting the corresponding acid with ammonium hydroxide solution or ammonia gas.

The following example describes the manner and process of making and using an anodizing electrolyte according to the present invention, and it sets forth the best mode contemplated by the inventors of carrying out the invention, but it is not to be construed as limiting.

### EXAMPLE

Tantalum powders suitable for use as an anode for an electrolytic capacitor are commercially available in two main types. Sodium reduction tantalum powders are available from H.C. Starck Inc., Newton, Mass. under the "NH" family designation. Beam melt tantalum powders are available from H.C. Starck Inc., Newton, Mass. under the "QR" family description. However, the present invention is not intended to be limited to these types of tantalum powders. Instead, the present invention is applicable to all types of valve metals and, in particular, all types of tantalum, whether in powder form or otherwise.

The anodizing breakdown voltage values of sintered tantalum pellets in several present invention electrolytes containing a mixture of water, polyethylene glycol 400, phosphoric acid and one of a group of a second acid (boric acid, acetic acid, adipic acid, azelaic acid, pimelic acid, 2,2-dimethylglutaric acid, trimethylacetic acid) were tested. In particular, the following electrolyte formulations were used:

The formation electrolytes consisted of: about 88% PEG 400 in water, about 0.8M of one of the second acids and the conductivity was adjusted to 120 µS/cm by phosphoric acid. In particular, a pre-mix of 88% PEG and 12% de-ionized water was made. Then 0.8M of each of the second acids was added and the conductivity was adjusted with H₃PO₄ to 120 µS/cm.

The anodes were individually formed in a jacketed beaker using 2.5-watt/gram protocols similar to that described in described in U.S. Patent No. 6,231,993 to Stephenson et al., which is assigned to the assignee of the present invention and incorporated herein by reference.

The current is initially set in a range of about 5 mA/gram to about 100 mA/gram amount of valve metal material during the formation process of either a sodium reduction or beam melt pressed valve metal powder structure. The formation voltage is then raised in intervals of about 10 volts to about 100 volts between periods when the formation voltage is turned off. The formation voltage is turned off for at least 10 minutes to allow for cooling and replenishment inside the anode pellet by fresh, relatively cool electrolyte having a conductivity more closely matching that of the anodization bath. It should also be pointed out that the current for each formation step preferably becomes less and less as the formation voltage increases. This is in order to keep the wattage from being too high. The formation protocol used in this example is a "constant wattage" protocol. The wattage increases with time at constant current within each formation step, but the maximum wattage for all formation steps is kept constant throughout the entire formation process. This requires the current to be reduced for each formation step as the formation voltage increases. The voltage intervals for each formation step preferably become less and less as the formation voltage gets closer to the target formation voltage. This is because as the formation voltage approaches the target formation voltage, the oxide coating on the anode pellet becomes thicker, which reduces heat dissipation and permeability of the pellet. Therefore, the voltage intervals are preferably decreased.

The formation protocol for a sodium reduced tantalum powder pellet was as follows. In this example, the pellet had a weight of about 8.3 grams and the target formation voltage was 500 volts. For each acid, three anodes were formed.
1. The power supply was turned on with a peak current of 279 mA to 75 volts. The power supply was then turned off for about half an hour.
2. The power supply was turned back on with a peak of 182 mA to 115 volts. The power supply was then turned off for about half an hour.
3. The power supply was turned back on with a peak of 144 mA to 145 volts. The power supply was then turned off for about half an hour.
4. The power supply was turned back on with a peak of 120 mA to 175 volts. The power supply was then turned off for about half an hour.
5. The power supply was turned back on with a peak of 105 mA to 200 volts. The power supply was then turned off for about half an hour.
6. The power supply was turned back on with a peak of 95 mA to 220 volts. The power supply was then turned off for about half an hour.
7. The power supply was turned back on with a peak of 87 mA to 240 volts. The power supply was then turned off for about half an hour.
8. The power supply was turned back on with a peak of 81 mA to 260 volts. The power supply was then turned off for about half an hour.
9. The power supply was turned back on with a peak of 75 mA to 280 volts. The power supply was then turned off for about half an hour.
10. The power supply was turned back on with a peak of 70 mA to 300 volts. The power supply was then turned off for about half an hour.
11. The power supply was turned back on with a peak of 65 mA to 320 volts. The power supply was then turned off for about half an hour.
12. The power supply was turned back on with a peak of 62 mA to 340 volts. The power supply was then turned off for about half an hour.
13. The power supply was turned back on with a peak of 58 mA to 360 volts. The power supply was then turned off for about half an hour.
14. The power supply was turned back on with a peak of 55 mA to 380 volts. The power supply was then turned off for about half an hour.
15. The power supply was turned back on with a peak of 52 mA to 400 volts. The power supply was then turned off for about half an hour.
16. The power supply was turned back on with a peak of 50 mA to 420 volts. The power supply was then turned off for about half an hour.
17. The power supply was turned back on with a peak of 48 mA to 440 volts. The power supply was then turned off for about half an hour.
18. The power supply was turned back on with a peak of 46 mA to 460 volts. The power supply was then turned off for about half an hour.
19. The power supply was turned back on with a peak of 44 mA to 480 volts. The power supply was then turned off for about half an hour.
20. The power supply was turned back on with a peak of 42 mA to 500 volts. The power supply was then turned off for about half an hour.

The anodized pellet was then rinsed and dried. This was followed by heat treat and reformation steps.

The formation protocol for a beam melt (BM) tantalum powder pellet was as follows. In this example, the pellet had a weight of about 4.4 grams and the desired target formation voltage was 500 volts. Three anodes were formed.
1. The power supply was turned on with a peak current of 147 mA to 75 volts. The power supply was then turned off for about half an hour.
2. The power supply was turned back on with a peak of 96 mA to 115 volts. The power supply was then turned off for about half an hour.
3. The power supply was turned back on with a peak of 76 mA to 145 volts. The power supply was then turned off for about half an hour.
4. The power supply was turned back on with a peak of 63 mA to 175 volts. The power supply was then turned off for about half an hour.
5. The power supply was turned back on with a peak of 55 mA to 200 volts. The power supply was then turned off for about half an hour.
6. The power supply was turned back on with a peak of 50 mA to 220 volts. The power supply was then turned off for about half an hour.
7. The power supply was turned back on with a peak of 46 mA to 240 volts. The power supply was then turned off for about half an hour.
8. The power supply was turned back on with a peak of 42 mA to 260 volts. The power supply was then turned off for about half an hour.
9. The power supply was turned back on with a peak of 39 mA to 280 volts. The power supply was then turned off for about half an hour.
10. The power supply was turned back on with a peak of 37 mA to 300 volts. The power supply was then turned off for about half an hour.
11. The power supply was turned back on with a peak of 34 mA to 320 volts. The power supply was then turned off for about half an hour.
12. The power supply was turned back on with a peak of 32 mA to 340 volts. The power supply was then turned off for about half an hour.
13. The power supply was turned back on with a peak of 31 mA to 360 volts. The power supply was then turned off for about half an hour.
14. The power supply was turned back on with a peak of 29 mA to 380 volts. The power supply was then turned off for about half an hour.
15. The power supply was turned back on with a peak of 28 mA to 400 volts. The power supply was then turned off for about half an hour.
16. The power supply was turned back on with a peak of 26 mA to 420 volts. The power supply was then turned off for about half an hour.
17. The power supply was turned back on with a peak of 25 mA to 440 volts. The power supply was then turned off for about half an hour.
18. The power supply was turned back on with a peak of 24 mA to 460 volts. The power supply was then turned off for about half an hour.
19. The power supply was turned back on with a peak of 23 mA to 480 volts. The power supply was then turned off for about half an hour.
20. The power supply was turned back on with a peak of 22 mA to 500 volts. The power supply was then turned off for about half an hour.

The anodized pellet was then rinsed and dried. This was followed by heat treat and reformation steps.

If desired, the formation process for either a sodium reduction or beam melt tantalum powder can be periodically interrupted and the anodized pellet subjected to a heat treatment step. This consists of removing the anode pellet from the anodization electrolyte bath. The anode pellet is then rinsed and dried followed by heat treatment according to the article by D. M. Smyth et al., "Heat-Treatment of Anodic Oxide Films on Tantalum", Journal of the Electrochemical Society, vol. 110, No. 12, pp. 1264-1271, December 1963. This publication is incorporated herein by reference.

Table 1 shows that the control electrolyte that contained only phosphoric acid broke down at 340V on the beam melt powder pellet and 265V on the sodium reduce powder pellet. However, all of the second acids either had comparable or higher breakdown voltages. Boric acid had the highest breakdown voltage among the acids tested.

**Table 1**

| | Formation Breakdown Voltage, V | |
|---|---|---|
| 2^{nd} Acid | BM powder pellet | Na reduced powder pellet |
| Phosphoric acid | 340 | 265 |
| Acetic Acid | 376 | 329 |
| Boric Acid | 500 | 329 |
| Azelaic Acid | 440 | 296 |
| Pimelic Acid | 337 | 325 |
| 2,2-Dimethylglutaric acid | 419 | 308 |
| Trimethylacetic acid | 345 | 298 |

Thus, the electrolytes of the present invention have high anodizing breakdown voltages and allow for higher voltage dielectric oxide formation. These properties are strongly desired for critical applications such as implantable cardioverter defibrillators (ICDs). When using in the ICDs, the capacitors made with anodes formed according to the present invention provide short first pulse charging time, high energy efficiency, and stable lifetime performance.

It is appreciated that various modifications to the present inventive concepts described herein may be apparent to those of ordinary skill in the art without departing from the spirit and scope of the present invention as defined by the herein appended claims.

## Claims

1. An anodizing electrolyte for providing a dielectric oxide on a valve metal, the anodizing electrolyte comprising:
a) water;
b) a phosphorus oxyacid or salt thereof; and
c) at least one of the group consisting of an inorganic acid, a salt of the inorganic acid, a carboxylic acid, a salt of the carboxylic acid, and mixtures thereof.

2. The anodizing electrolyte of claim 1 wherein the water is present in a range of from about 5% to about 80%, by weight.

3. The anodizing electrolyte of claim 1 or claim 2 wherein the phosphorus oxyacid is in a range of from about 0.1% to about 15%, by weight.

4. The anodizing electrolyte of any of claims 1 to 3 wherein the phosphorus oxyacid is selected from the group consisting of phosphoric acid, hypophosphoric acid, phosphorous acid, hypophosphorous acid, metaphosphoric acids, and mixtures thereof.

5. The anodizing electrolyte of any of claims 1 to 4 wherein the phosphorus oxyacid salt is selected from the group consisting of ammonium dihydrogen phosphate, ammonium hydrogen phosphate, sodium phosphates, potassium phosphates, and mixtures thereof.

6. The anodizing electrolyte of any of claims 1 to 5 wherein the inorganic acid or the carboxylic acid is in a range of from about 0.5% to about 15%, by weight.

7. The anodizing electrolyte of any of claims 1 to 6 wherein the inorganic acid is boric acid and the inorganic acid salt is selected from the group consisting of ammonium borate, sodium borate, ammonium tetraborate, ammonium pentaborate, and mixtures thereof.

8. The anodizing electrolyte of any of claims 1 to 7 wherein the carboxylic acid is a mono-carboxylic acid having from 2 to 7 carbon atoms.

9. The anodizing electrolyte of any of claims 1 to 7 wherein the carboxylic acid is a di-carboxylic acid or a poly-carboxylic acid having from 3 to 13 carbon atoms.

10. The anodizing electrolyte of any of claims 1 to 7 wherein the carboxylic acid is selected from the group consisting of acetic acid, propanoic acid, butyric acid, iso-butyric acid, trimethyl acetic acid, cyclohexanecarboxylic acid, dicyclohexylacetic acid, malonic acid, glutaric acid, adipic acid, pimelic acid, azelaic acid, brassylic acid, dodecanedioic acid, 1,2-cyclohexanedicarboxylic acid, cyclohexyldicarboxylic acid, methylmalonic acid, dimethylmalonic acid, 2,2-dimethylsuccinic acid, 2-methylglutaric acid, 2,2-dimethylglutaric acid, 3-(tert-butyl)adipic acid, citric acid, tartaric acid, 1,3,5-cyclohexanetricarboxylic acid, and mixtures thereof.

11. The anodizing electrolyte of any of claims 1 to 10 wherein the valve metal is selected from the group consisting of tantalum, vanadium, niobium, aluminum, titanium, zirconium, hafnium, and mixtures thereof.

12. The anodizing electrolyte of any of claims 1 to 11 wherein the electrolyte further includes a protic solvent.

13. The anodizing electrolyte of claim 12 wherein the protic solvent is present in at up to about 90%, by weight.

14. The anodizing electrolyte of claim 12 or claim 13 wherein the protic solvent is selected from the group consisting of alkylene glycols, polyalkylene glycols, alkylene glycol monoethers, polyalkylene glycol monoethers, and mixtures thereof.

15. The anodizing electrolyte of any of claims 12 to 14 wherein the protic solvent is selected from the group consisting of ethylene glycol, diethylene glycol, triethylene glycol, tetraethylene glycol, propylene glycol, trimethylene glycol, dipropylene glycol, glycerol, 2-methyl-1,3-propanediol, 1,4-butanediol, 2,3-butanediol, 2,2-dimethyl-1,3-propanediol, 2,4-pentanediol, 2,5-hexanediol, polyethylene glycols, polypropylene glycols, polyethylenepropylene glycol copolymers, ethylene glycol methyl ether, ethylene glycol ethyl ether, ethylene glycol butyl ether, diethylene glycol ethyl ether, diethylene glycol methyl ether, diethylene glycol butyl ether, dipropylene glycol methyl ether, tripropylene glycol methyl ether, and mixtures thereof.

16. The anodizing electrolyte of any of claims 1 to 15 having a conductivity of about 20 µS to about 10,000 µS at 40°C.

17. The anodizing electrolyte of any of claims 1 to 16 having a conductivity of about 100 µS to about 1,000 µS at 40°C.

18. An anodizing electrolyte for providing a dielectric oxide on a valve metal, the anodizing electrolyte consisting essentially of:
a) water;
b) a phosphorus oxyacid; and
c) a second acid selected from the group consisting of boric acid, acetic acid, propanoic acid, butyric acid, iso-butyric acid, trimethyl acetic acid, cyclohexanecarboxylic acid, dicyclohexylacetic acid, malonic acid, glutaric acid, adipic acid, pimelic acid, azelaic acid, brassylic acid, dodecanedioic acid, 1,2-cyclohexanedicarboxylic acid, cyclohexyldicarboxylic acid, methylmalonic acid, dimethylmalonic acid, 2,2-dimethylsuccinic acid, 2-methylglutaric acid, 2,2-dimethylglutaric acid, 3-(tert-butyl)adipic acid, citric acid, tartaric acid; 1,3,5-cyclohexanetricarboxylic acid, and mixtures thereof.

19. The anodizing electrolyte of claim 18 wherein the water is present in a range of from about 5% to about 80%, the phosphorus oxyacid is in a range of from about 0.1% to about 15%, and the second acid is in a range of from about 0.5% to about 15%, by weight.

20. The anodizing electrolyte of claim 18 or claim 19 having a conductivity of about 20 µS to about 10,000 µS at 40°C.

21. A method for anodizing a valve metal structure, comprising the steps of:
a) providing the valve metal structure selected from the group consisting of tantalum, vanadium, niobium, aluminum, titanium, zirconium, hafnium, and mixtures thereof;
b) providing an anodizing electrolyte comprising water, a phosphorus oxyacid, and a second acid selected from the group consisting of boric acid, acetic acid, propanoic acid, butyric acid, iso-butyric acid, trimethyl acetic acid, cyclohexanecarboxylic acid, dicyclohexylacetic acid, malonic acid, glutaric acid, adipic acid, pimelic acid, azelaic acid, brassylic acid, dodecanedioic acid, 1,2-cyclohexanedicarboxylic acid, cyclohexyldicarboxylic acid, methylmalonic acid, dimethylmalonic acid, 2,2-dimethylsuccinic acid, 2-methylglutaric acid, 2,2-dimethylglutaric acid, 3-(tert-butyl)adipic acid, citric acid, tartaric acid, 1,3,5-cyclohexanetricarboxylic acid, and mixtures thereof; and
c) applying a current to the valve metal immersed in the anodizing electrolyte until a target formation voltage is reached.

22. The method of claim 21 including providing the valve metal structure being selected from the group consisting of an etched foil, an unetched foil, and a sintered powder body.

23. The method of claim 21 or claim 22 including periodically turning off the formation current and letting the valve metal structure rest in the anodizing electrolyte.

24. The method of claim 23 including turning off the formation current for at least 10 minutes.

25. The method of any of claims 21 to 24 including providing the current in a range of from about 5 mA/gram to about 100 mA/gram amount of the valve metal.

26. The method of any of claims 21 to 25 including raising a formation voltage in intervals of from about 10 volts to about 100 volts toward the target formation voltage between periodically turning off the formation current and letting the valve metal structure rest in the anodizing electrolyte.

27. The method of any of claims 21 to 26 including anodizing the valve metal structure using a constant wattage protocol where wattage increases with time at constant current within each formation step between when the formation current is turned off, but the maximum wattage for each formation step is kept constant throughout the entire formation process.

28. The method of any of claims 21 to 27 including providing the anodizing electrolyte having the water in a range of from about 5% to about 80%, the phosphorus oxyacid in a range of from about 0.1% to about 15%, and the second acid in a range of from about 0.5% to about 15%, by weight.

29. The method of any of claims 21 to 28 including providing the electrolyte having a conductivity of about 20 µS to about 10,000 µS at 40°C.
